# EUROPÄISCHE PATENTSCHRIFT

(11) **EP 1 663 993 B1**
(45) Veröffentlichungstag und Bekanntmachung des Hinweises auf die Patenterteilung: **12.03.2008**
(21) Anmeldenummer: 04761896.2
(22) Anmeldetag: 03.09.2004
(51) Int. Cl.: C07D 239/84, A61K 31/517, A61P 29/00

(54) **GUANIDINDERIVATE**
GUANIDINE DERIVATIVES
DERIVES DE GUANIDINE

(30) Priorität: 05.09.2003 CH 152603
(43) Veröffentlichungstag der Anmeldung: 07.06.2006
(73) Patentinhaber: Actelion Pharmaceuticals Ltd., 4123 Allschwil (CH)
(72) Erfinder: FECHER, Anja, 4059 Basel (CH); FRETZ, Heinz, CH-4125 Riehen (CH); HILPERT, Kurt, CH-4114 Hofstetten (CH); BREU, Volker, 79418 Schliengen (DE); GILLER, Thomas, CH-4451 Wintersingen (CH); VALDENAIRE, Olivier, CH-4123 Allschwil (CH)
(74) Vertreter: Ruhlmann, Eric
(86) Internationale Anmeldenummer: PCT/CH2004/000556
(87) Internationale Veröffentlichungsnummer: WO 2005/023781

(56) Entgegenhaltungen:
- WO-A-03/026667
- US-A1- 2003 139 431

## Beschreibung

Die vorliegende Erfindung betrifft Guanidinderivate der allgemeinen Formel 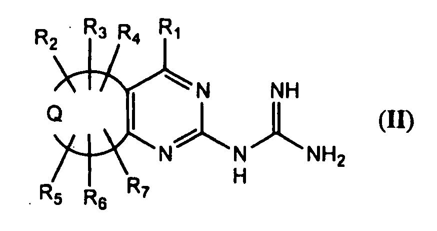 worin
R₁ Methyl, Ethyl, Trifluormethyl, Methylamino, Ethylamino, Isopropylamino, Cyclopropylamino, Methoxy, Ethoxy, Trifluormethoxy, Methylsulfanyl, oder Ethylsulfanyl bedeutet;
Q eine Kette von 3-6 gegebenenfalls substituierten C-Atomen bedeutet, wovon eines oder mehrere durch -N(R')-, -O- oder -S(O)ₘ ersetzt sein können, wobei im Falle mehrerer solcher Atome oder Gruppen diese gleich oder verschieden sein können, wobei Q zusammen mit dem Pyrimidinring ein Chinazolin-, Cyclopentapyrimidin-, Cycloheptapyrimidin, Pyridopyrimidin-, Pyranopyrimidin-, Thiopyranopyrimidin-, Pyrimidoazepin- oder Cyclooctapyrimidingerüst bildet, welches nur die drei Doppelbindungen des Pyrimidinbausteins enthält;
R' Methyl, Ethyl, Propyl, Hexyl, 2,2-Dimethylpropionyl, Cyclopropylmethyl, 2-Cyclohexylethyl, Propinyl, Ethyloxycarbonylethyl, Benzyl, n-Butyloxycarbonyl, tert-Butyloxycarbonyl, Benzyloxycarbonyl, 3-Methyl-butyryl, Pentanoyl, Phenylacetyl, 2-Propylpentanoyl, Cyclopropancarbonyl, Isobutyryl, But-3-enoyl, 2-Methoxyacetyl, Propane-2-sulfonyl, Butane-1-sulfonyl, Methansulfonyl, tert-Butyloxycarbonylaminopropionyl oder 4-Dimethylamino-butyryl bedeutet;
R₂ Methyl, Ethyl, n-Propyl, Isopropyl, n-Butyl, Isobutyl, tert.-Butyl, 1,1-Dimethylpropyl oder Phenyl bedeutet; R₃-R₇ Wasserstoff, Methyl, Ethyl, n-Propyl, i-Propyl, n-Butyl oder t-Butyl bedeuten; und
m 0, 1 oder 2 bedeutet;
pharmazeutisch verwendbare Säureadditionssalze von basischen Verbindungen der Formel **II,** pharmazeutisch verwendbare Salze von saure Gruppen enthaltenden Verbindungen der Formel **II** mit Basen, pharmazeutisch verwendbare Ester von Hydroxy- oder Carboxygruppen enthaltenden Verbindungen der Formel **II,** sowie Hydrate oder Solvate davon.

Diese Verbindungen sind neu, und sie zeichnen sich durch wertvolle pharmakodynamische Eigenschaften aus. Sie wirken als Neuropeptid FF Rezeptor-Antagonisten und eignen sich zur Behandlung von Schmerz, für die Kontrolle von Schmerzüberempfindlichkeit (Hyperalgesie), von chronischen, akuten, lang andauernden, vorübergehenden Schmerzen, wobei diese Schmerzen operativen, traumatischen, oder pathologischen Ursprungs sein können, mit dem Vorteil, Opioidtoleranz und/oder Opioidabhängigkeit zu verhindern oder rückgängig zu machen. Die erfindungsgemässen Stoffe sind zudem zur Behandlung von Entzugserscheinungen bei Alkohol-, Psychopharmaka- und Nicotinabhängigkeit und zur Verhinderung oder Aufhebung dieser Abhängigkeiten geeignet. Zusätzlich können die Verbindungen zur Regulierung der Insulin-Freisetzung, der Nahrungsaufnahme, von Gedächtnisfunktionen, des Blutdrucks, des Elektrolyt- und Energiehaushaltes, sowie bei der Behandlung von Harninkontinenz eingesetzt werden.

Guanidinderivate der Formel **II**, welche ein oder mehrere Asymmetriezentren enthalten, können als optisch reine Enantiomere, als Mischungen von Enantiomeren, wie zum Beispiel Racemate, oder gegebenenfalls als optisch reine Diastereomere, als Mischungen von Diastereomeren, als diastereomere Racemate oder als Mischungen von diastereomeren Racematen vorliegen.

Die Neuropeptide FF (NPFF), AF (NPAF), SF (NPSF) und VF (NPVF) sind verwandte Neurotransmittoren mit schmerzmodulierenden Eigenschaften. Sie bilden zusammen mit den kürzlich entdeckten G-Protein gekoppelten Rezeptoren, NPFF1 und NPFF2, einen wichtigen Teil eines endogenen Systems, welches die Schmerzempfindung in verschiedenen Säugerarten wie Mensch, Ratte, Maus, Rind usw. reguliert. Die erwähnten Neuropeptide scheinen sowohl bei der opioidabhängigen Analgesie als auch der Entwicklung von Toleranz gegenüber Opioiden eine wichtige Rolle zu spielen (Übersichtsartikel: Roumy and Zajac, Europ. J. Pharm. 1998, 345, 1-11; Panula et al., Prog. Neurobiol. 1996, 48, 461-87). Weiteren Berichten zufolge scheint NPFF auch in physiologischen Vorgängen wie Insulin-Freisetzung, Regulierung der Nahrungsaufnahme, von Gedächtnisfunktionen, des Blutdruckes und des Elektrolythaushaltes eine Rolle zu spielen (Panula et. al., Prog. Neurobiol. 1996, 48, 461-487).

Das Vorkommen von funktionalen NPFF1 und NPFF2 Rezeptoren in Adipocyten und die Wirkung von NPFF und NPAF auf Schlüsselstellen der Signalübertragung im adiposen Metabolismus deuten darauf hin, dass die beiden Peptide neben ihren ursprünglichen schmerzmodulierenden Effekten, zusätzlich einen Einfluss auf Speicherung und Verwendung von Körperenergie haben könnten (Lefrere et al., J. Biol. Chem. 2002, 277 (42), 39169).

Die gängigen Behandlungsmöglichkeiten von chronischem Schmerz basieren auf NSAIDs (non-steroidal antiinflammatory drugs), Canabinoiden und Opioiden. So binden zum Beispiel Morphinderivate an den µ-opioiden Rezeptor und wirken dadurch schmerzlindernd. Opioidbindung an den µ-opioiden Rezeptor geht einher mit der Freisetzung von Neuropeptid FF. Basierend auf Tierexperimenten wird vermutet, dass das freigesetzte NPFF den analgetischen Effekt der verabreichten Opioide abschwächt und zu Toleranz gegenüber Opioiden führt. Um bei längeren Behandlungen einen gleichbleibenden schmerzlindernden Effekt zu erhalten, müssen infolge dieser Toleranz zunehmend höhere Opioid-Dosen verabreicht werden, was schliesslich zu ernsthaften Nebenwirkungen führen kann. Wie eingangs schon erwähnt, sind bis heute zwei Neuropeptid FF Rezeptoren bekannt, wobei der NPFF1-Rezeptor hauptsächlich im zentralen Nervensystem und der NPFF2-Rezeptor vornehmlich im Rückenmark lokalisiert ist. Aktivieren der NPFF2-Rezeptoren zeigt eine opioid-ähnliche analgetische Wirkung. Blockieren der NPFFI-Rezeptoren durch einen Antagonisten verhindert die Entwicklung von Toleranz gegenüber Opioiden und erhöht zusätzlich deren Wirkung.

Kawakami J. K. et al. (PCT Application WO03/026667, publiziert am 3. April 2003) berichten von Chinazolinguanidin- und Chinolinguanidin-Derivaten als NPFF-Rezeptor Liganden.

Wie eingangs erwähnt sind die erfindungsgemässen Stoffe neu und zeichnen sich durch wertvolle pharmakologische Eigenschaften aus. Aufgrund ihrer Eigenschaft, die Interaktion von Neuropeptid FF mit dem Neuropeptid FF1-Rezeptor-Subtyp zu blockieren, eignen sich die erfindungsgemässen Verbindungen der Formel II und deren pharmazeutisch verwendbare Salze zur Verwendung als Arzneimittel, insbesondere für die Behandlung von Schmerz und Hyperalgesie, wobei die erfindungsgemässen Stoffe die gängigen Behandlungsmethoden von chronischen Schmerzen ergänzen, und zwar mit dem Vorteil, die unerwünschte Opioidtoleranz und/oder Abhängigkeit zu verhindern oder rückgängig zu machen. Die erfindungsgemässen Stoffe sind zudem zur Behandlung von Entzugserscheinungen bei Alkohol-, Psychopharmaka- und Nicotinabhängigkeit und zur Verhinderung oder Aufhebung dieser Abhängigkeiten geeignet. Zusätzlich können sie zur Regulierung der Insulin-Freisetzung, der Nahrungsaufnahme, von Gedächtnisfunktionen, des Blutdruckes, des Elektrolyt- und Energiehaushaltes, sowie bei der Behandlung von Harninkontinenz eingesetzt werden.

Gegenstand der vorliegenden Erfindung sind die neuen Stoffe als solche und als therapeutische Wirkstoffe; Verfahren und Zwischenprodukte zu deren Herstellung; Arzneimittel, enthaltend einen der obigen Stoffe; die Herstellung solcher Arzneimittel und die Verwendung der obigen Stoffe zur Vorbeugung und Behandlung von Schmerzüberempfindlichkeit (Hyperalgesie), von chronischen, akuten, lang andauernden, vorübergehenden Schmerzen, welche operativen, traumatischen, oder pathologischen Ursprungs sein können, von Entzugserscheinungen bei Alkohol-, Psychopharmaka- und Nicotinabhängigkeit und zur Verhinderung oder Aufhebung dieser Abhängigkeiten, zur Regulierung der Insulin-Freisetzung, der Nahrungsaufnahme, von Gedächtnisfunktionen, des Blutdrucks, des Elektrolyt- und Energiehaushaltes, und zur Behandlung von Harninkontinenz bzw. zur Herstellung entsprechender Arzneimittel.

Bevorzugte Bedeutungsmöglichkeiten für R₁ sind Methyl und Trifluormethyl.

Wenn eines oder mehrere der C-Atome in der Kette Q in Formel **II** substituiert ist/sind, dann kann
- eines der C-Atome einen oder zwei (also geminale) gleiche oder verschiedene Substituenten tragen; oder es können
- mehrere der C-Atome je einen oder zwei (also geminale) gleiche oder verschiedene Substituenten tragen.

In Formel **II** kann Q zusammen mit einem Pyrimidinring zum Beispiel ein 6,7-Dihydro-5H-cyclopentapyrimidin-, 5,6,7,8-Tetrahydro-chinazolin-, 6,7,8,9-Tetrahydro-5H-cycloheptapyrimidin-, 5,6,7,8,9,10-Hexahydrocyclooctapyrimidin-, 6,7-Dihydro-5H-pyrrolopyrimidin- oder 5,6,7,8-Tetrahydro-pyridopyrimidingerüst bilden.

Ganz besonders bevorzugte Verbindung der Formel **II** sind
N-(4-Methyl-6-propyl-5,6,7,8-tetrahydro-chinazolin-2-yl)-guanidin;
N-(6-Isopropyl-4-methyl-5,6,7,8-tetrahydro-chinazolin-2-yl)-guanidin;
N-(4-Methyl-5,6,7,8-tetrahydro-chinazolin-2-yl)-guanidin;
N-(4,5-Dimethyl-5,6,7,8-tetrahydro-chinazolin-2-yl)-guanidin und N-(6-tert-Butyl-4-methyl-5,6,7,8-tetrahydro-chinazolin-2-yl)-guanidin.

Weitere besonders bevorzugte Verbindungen der Formel **II** sind
N-(4-Methyl-8-phenyl-5,6,7,8-tetrahydro-chinazolin-2-yl)-guanidin;
N-(4-Methyl-6-phenyl-5,6,7,8-tetrahydro-chinazolin-2-yl)-guanidin;
N-[6-(1,1-Dimethyl-propyl)-4-methyl-5,6,7,8-tetrahydrochinazolin-2-yl]-guanidin;
N-(8-tert-Butyl-4-methyl-5,6,7,8-tetrahydro-chinazolin-2-yl)-guanidin;
N-(4,6-Dimethyl-5,6,7,8-tetrahydro-chinazolin-2-yl)-guanidin;
N-(4-Methyl-6,7,8,9-tetrahydro-5H-cycloheptapyrimidin-2-yl)-guanidin; und
N-(4-Methyl-5,6,7,8,9,10-hexahydro-cyclooctapyrimidin-2-yl)-guanidin.

Ebenfalls bevorzugte Verbindungen der Formel **II** sind
N-(4,8-Dimethyl-5,6,7,8-tetrahydro-chinazolin-2-yl)-guanidin;
N-(6-tert-Butyl-4-trifluoromethyl-5,6,7,8-tetrahydro-chinazolin-2-yl)-guanidin.

Weitere repräsentative Verbindungen der Formel **II** sind auch
2-Guanidino-4-methyl-7,8-dihydro-5H-pyrido[4,3-d]pyrimidin-6-carbonsäure-tert-butylester;
N-(6-Phenyl-4-trifluormethyl-5,6,7,8-tetrahydro-chinazolin-2-yl)-guanidin; und
N-(6-Isopropyl-4-trifluormethyl-5,6,7,8-tetrahydrochinazolin-2-yl)-guanidin.

Die erfindungsgemässen Verbindungen der Formel **II** können in freier Form, als pharmazeutisch verwendbare Säureadditionssalze, als pharmazeutisch verwendbare Salze von sauren Verbindungen der Formel **II** mit Basen, als pharmazeutisch verwendbare Ester von Hydroxy- oder Carboxygruppen enthaltenden Verbindungen der Formel **II** sowie als Hydrate oder Solvate davon vorliegen. Der Begriff "pharmazeutisch verwendbare Salze" bezieht sich auf solche Salze, welche die biologische Wirkung und Eigenschaften der freien Basen nicht mindern und welche nicht biologisch oder anderweitig unerwünscht sind.

Die Säureadditionssalze werden aus den freien Basen mittels anorganischer Säuren, wie Salzsäure, Bromwasserstoffsäure, Schwefelsäure, Salpetersäure, Phosphorsäure und dgl., vorzugsweise Salzsäure oder Bromwasserstoffsäure, oder mittels organischer Säuren, wie Essigsäure, Propionsäure, Glycolsäure, Brenztraubensäure, Oxalsäure, Maleinsäure, Malonsäure, Bernsteinsäure, Weinsäure, Salicylsäure, Citronensäure, Benzoesäure, Mandelsäure, Methansulfonsäure, p-Toluolsulfonsäure und dgl. gebildet. Falls bestimmte Verbindungen der Formel **II** durch die weiter unten beschriebene Cycloaddition von Bisguanidincarbonat hergestellt werden, können sie als Carbonate anfallen.

Verbindungen der Formel **II,** die saure Gruppen enthalten, können mit anorganischen oder organischen Basen Salze bilden. Bevorzugte Salze mit anorganischen Basen sind, aber nicht ausschliesslich, Natrium-, Kalium-, Lithium-, Ammonium-, Calcium-, Magnesiumsalze und dgl. Bevorzugte Salze mit organischen Basen sind, aber nicht ausschliesslich, Salze mit primären, sekundären und tertiären, gegebenenfalls substituierten Aminen, einsch-liesslich alle natürlich vorkommenden substituierten Amine, mit cyclischen Aminen und basischen Ionenaustauschharzen, wie Isopropylamin, Trimethylamin, Diethylamin, Triethylamin, Tripropylamin, Ethanolamin, Lysin, Arginin, N-Ethylpiperidin, Piperidin, Polyamin-Harze und dgl. Verbindungen der Formel **II,** die eine saure Gruppe tragen, können auch als Zwitterionen vorliegen.

Die Erfindung umfasst auch pharmazeutisch geeignete Ester von Hydroxy- oder Carboxygruppen enthaltenden Verbindungen der Formel **II.** "Pharmazeutisch geeignete Ester" bedeutet, dass in Verbindungen der Formel **II** entsprechende funktionelle Gruppen so zu Estergruppen derivatisiert sind, dass sie in vivo wieder in ihre aktive Form zurücktransformiert werden. Einerseits können COOH-Gruppen verestert sein. Beispiele geeigneter derartiger Ester sind die Alkyl- und Aralkylester. Bevorzugte derartige Ester sind Methyl-, Ethyl-, Propyl-, Butyl- und Benzylester sowie (R/S)-1-[(Isopropoxycarbonyl)oxy]ethylester. Besonders bevorzugt sind Ethylester und die isomeren Butylester. Anderseits können OH-Gruppen verestert sein. Beispiele solcher Verbindungen enthalten physiologisch akzeptable und metabolisch labile Estergruppen, wie Methoxymethylester-, Methylthiomethylester-, Pivaloyloxymethylester- und ähnliche Estergruppen.

Verbindungen der Formel **II** wurden im folgenden Test auf ihre Affinität für die NPFF Rezeptoren untersucht:

Für Neuropeptid FF Rezeptor-Bindungsstudien geeignete Hamster-Zellen (Chinese Hamster Ovary cells, CHOSP10), welche jeweils den NPFF1 oder NPFF2 Rezeptor produzieren, wurden bei Standard Zellkulturbedingungen vermehrt. Das Zellkulturmedium wurde abgesaugt und 5 ml von Puffer A (5 mM Tris pH=7.4, 1 mM MgCl₂) pro 17cm Petrischale zugegeben. Die Zellen wurden von der Zellkulturplatte abgeschabt und in ein 50 ml Falkon-Gefäss transferiert. Danach wurden die Zellen 5 Minuten bei 450 g zentrifugiert, wiederum im Puffer A resuspendiert und 30 Sekunden auf einem Polytron Vortexer gemixt. Nach einer Zentrifugation bei 30'000 g während 20 min. wurde der Überstand verworfen und das Membranpellet in 500 µl Puffer C (75 mM Tris PH=7.4, 25 mM MgCl₂, 250 mM Saccharose, 0.1 mM PMSF, 0.1 mMm Phenanthrolin) aufgenommen. Das Membran-Puffer-Gemisch wurde danach in Aliquots aufgeteilt und tiefgefroren. Der Proteingehalt von einem Aliquot wurde nach der Methode von Lowry bestimmt.

Der Bindungstest wurde in einem Endvolumen von 250 µl durchgeführt. 100 µl Membran-Puffermix entsprechend 35 µg Proteingehalt wurden mit 95 µl Bindungs-Puffer (50 mM Tris pH 7.4, 60 mM NaCl, 0.1 % Protease freies BSA, 0.01% NaN₃) gemischt. Nach Zugabe von 5 µl jeweils einer Konzentration Testsubstanz pro Messpunkt, wurde 0.2 nM ¹²⁵I-Tyr1-NPFF (NEN, NEX381) pro Messpunkt in 50 µl zugegeben. Nach 90 min. Inkubation bei Zimmertemperatur wurden die Proben durch ein GF/C Filter (Millipore (MAHFC1H60)) abgesaugt und der Filter mit eiskaltem Bindungs-Puffer mit dreimal 300 µl gewaschen (Packard Filtermate). Nach Zugabe von 55 µl Microscint 40 (Packard 6013641) Scintillationsflüssigkeit wurden die Messpunkte im Gamma-Counter (Packard, Top Count NXT) quantifiziert.

Nichtspezifische Bindung wurde in Gegenwart von 1 µM unmarkiertem Neuropeptid FF ermittelt. Spezifische Bindung ist definiert als die Differenz zwischen totaler und nichtspezifischer Bindung. IC₅₀ Werte werden definiert als diejenige Konzentration des Antagonisten, welche 50% des ¹²⁵ I-markierten Neuropeptid FF verdrängt. Diese Konzentration wird durch lineare Regressions-Analyse nach logit/log-Transformation der Bindungswerte ermittelt.

Bevorzugte erfindungsgemässe Verbindungen zeigen in der oben beschriebenen Rezeptor Bindungsstudie IC₅₀ Werte unter 1000 nM, besonders bevorzugte Verbindungen zeigen IC₅₀ Werte unter 100 nM, ganz besonders bevorzugte unter 10 nM.

Die Ergebnisse von im vorstehend beschriebenen biologischen Test untersuchten repräsentativen Verbindungen der Formel **II** sind in der nachfolgenden Tabelle 1 zusammengestellt.

**Tabelle 1: NPFF1-Rezeptor Bindung**

| **Verbindung** | **Binding NPFF1 IC₅₀ [nM]** |
|---|---|
| rac-N-(4-Methyl-6-propyl-5,6,7,8-tetrahydro-chinazolin-2-yl)-guanidin | 1 |
| rac-N-(6-Isopropyl-4-methyl-5,6,7,8-tetrahydro-chinazolin-2-yl)-guanidin | 2 |
| rac-N-(4-Methyl-5,6,7,8-tetrahydro-chinazolin-2-yl)-guanidin | 4 |
| rac-N-(4,5-Dimethyl-5,6,7,8-tetrahydro-chinazolin-2-yl)-guanidin | 7 |
| rac-N-(6-tert-Butyl-4-methyl-5,6,7,8-tetrahydro-chinazolin-2-yl)-guanidin | 8 |
| rac-N-(4-Methyl-8-phenyl-5,6,7,8-tetrahydro-chinazolin-2-yl)-guanidin | 16 |
| rac-N-(4-Methyl-6-phenyl-5,6,7,8-teträhydro-chinazölin-2-yl)-guanidin | 16 |
| rac-N-[6-(1,1-Dimethyl-propyl)-4-methyl-5,6,7,8-tetrahydro-chinazolin-2-yl]-guanidin | 19 |
| rac-N-(8-tert-Butyl-4-methyl-5,6,7,8-tetrahydro-chinazolin-2-yl)-guanidin . | 39 |
| rac-N-(4,6-Dimethyl-5,6,7,8-tetrahydro-chinazolin-2-yl)-guanidin | 48 |
| rac-N-(4-Methyl-6,7,8,9-tetrahydro-5H-cycloheptapyrimidin-2-yl)-guanidin | 54 |
| rac-N-(4-Methyl-5,6,7,8,9,10-hexahydro-cyclooctapyrimidin-2-yl)-guanidin | 60 |

Wie eingangs erwähnt, sind die erfindungsgemässen Verbindungen aufgrund ihrer Fähigkeit, die Neuropeptid FF Rezeptoren zu blockieren, wertvoll zur Behandlung von Schmerz, von Schmerzüberempfindlichkeit (Hyperalgesie) und von chronischen, akuten, lang andauernden oder vorübergehenden Schmerzen, wobei diese Schmerzen operativen, traumatischen, oder pathologischen Ursprungs sein können. Vor allem ergänzen sie die gängigen Behandlungsmethoden von chronischen Schmerzen mit dem Vorteil, die unerwünschte Opioidtoleranz und/oder Abhängigkeit zu verhindern oder rückgängig zu machen. Die erfindungsgemässen Stoffe sind zudem zur Behandlung von Entzugserscheinungen bei Alkohol-, Psychopharmaka- und Nicotinabhängigkeit und zur Verhinderung oder Aufhebung dieser Abhängigkeiten geeignet. Zusätzlich können die Verbindungen zur Regulierung der Insulin-Freisetzung, der Nahrungsaufnahme, von Gedächtnisfunktionen, des Blutdrucks des Elektrolyt- und Energiehaushaltes, sowie zur Behandlung von Inkontinenz eingesetzt werden.

Die erfindungsgemässen Verbindungen können nach allgemein bekannten und jedem Fachmann geläufigen Methoden in geeignete galenische Darreichungsformen gebracht werden. Solche Darreichungsformen sind beispielsweise Tabletten, Lacktabletten, Dragees, Kapseln, Injektionslösungen usw. Zur Herstellung solcher galenischer Darreichungsformen geeignete Exzipientien und Hilfsstoffe sind ebenfalls allgemein bekannt und jedem Fachmann geläufig. Ausser einer oder mehreren erfindungsgemässen Verbindungen können diese Darreichungsformen auch noch weitere pharmakologisch aktive Verbindungen enthalten.

Die Dosierung der erfindungsgemässen Verbindungen bzw. der sie enthaltenden Darreichungsformen ist vom behandelnden Arzt den jeweiligen Bedürfnissen des Patienten anzupassen. Im Allgemeinen dürfte eine Tagesdosis von 0.1-20 mg, bevorzugt 0.5-5 mg einer erfindungsgemässen Verbindung pro kg Körpergewicht des Patienten angebracht sein.

Die erfindungsgemässen Guanidinderivate der allgemeinen Formel **II**, sowie die entsprechenden Ausgangs- und Zwischenprodukte können mittels in der organischen Synthese bekannter Methoden hergestellt und unter Verwendung bekannter Techniken wie Fällen, Chromatografieren, Kristallisieren, präperative *reverse-phase* HPLC, usw. isoliert und gereinigt werden. Allfällig erhaltenene Stereoisomerengemische, wie Racemate, können nach allgemein üblichen Methoden aufgetrennt werden, bevorzugt durch Chromatografieren an einer chiralen Phase.

Allgemein können bicyclische, Guanidingruppen enthaltende Verbindungen der Formel **II** gemäss dem nachstehenden Schema 1 hergestellt werden:

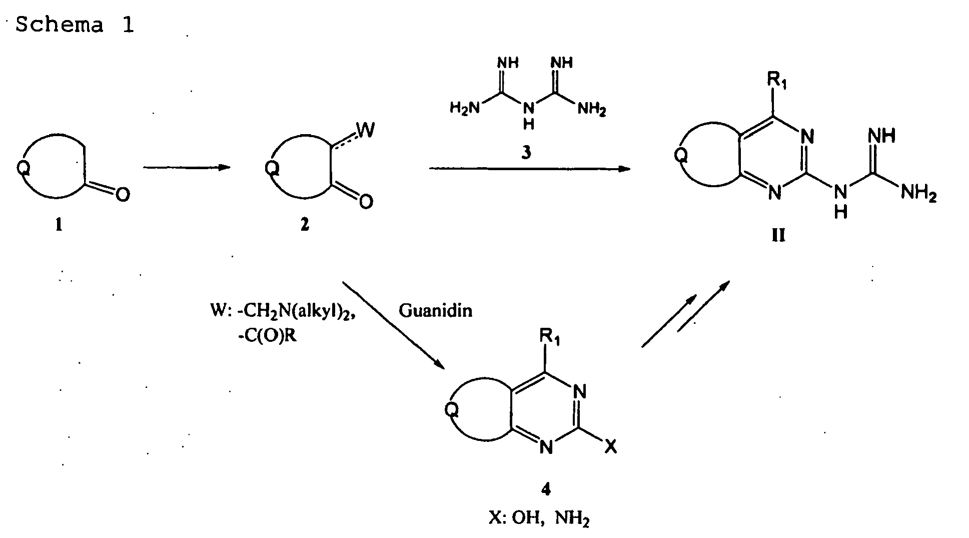

Eine Verbindung der Formel **1,** worin das oder die in Q allfällig vorhandene(n) Stickstoffatom(e) geschützt oder mit einem einen Rest R' abgebenden Mittel entsprechend substituiert ist/sind, wird in α-Stellung zur Carbonylgruppe nach bekannten Methoden mit einer Funktion W aktiviert, z.B. acyliert, formyliert, oder aminoalkyliert, worauf man die erhaltene Verbindung der Formel **2** mit einem stickstoffhaltigen Reagens, wie Bisguanidin der Formel **3** oder Guanidin einer Cyclokondensation unterwirft, gegebenenfalls die erhaltene Verbindung der Formel **4** mittels an sich bekannter Methoden in die Zielverbindung der Formel **II** überführt, gegebenenfalls aus der erhaltenen Verbindung die an dem oder den allfällig vorhandenen Stickstoffatom(en) sitzende(n) Schutzgruppe(n) abspaltet, gegebenenfalls diese(s) Stickstoffatom(e) mit einem / einen Rest R' abgebenden Mittel entsprechend substituiert und gegebenenfalls eine erhaltene basische Verbindung in ein pharmazeutisch verwendbares Salz mit einer Säure, bzw. eine erhaltene, eine saure Gruppe enthaltende basische Verbindung in ein pharmazeutisch verwendbares Salz mit einer Base bzw. eine erhaltene, Hydroxy- oder Carboxygruppen enthaltende Verbindung in einen pharmazeutisch verwendbaren Ester überführt und gegebenenfalls das erhaltene Produkt in ein Hydrat oder Solvat überführt.

So lassen sich bicyclische Pyrimidin-Derivate der Formel **IV,** welche eine Untergruppe der Verbindungen der Formel **II** darstellen, gemäss dem nachstehenden Schema 2 herstellen:

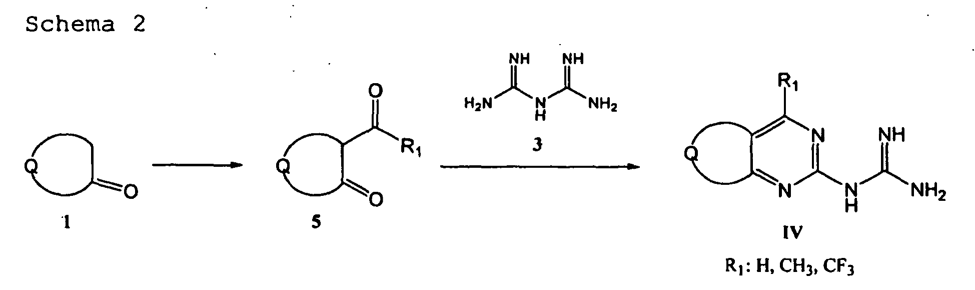

Cycloalkanone der Formel **1** können mit bekannten Methoden in Position α zur Carbonylgruppe acyliert (J. Med. Chem. 1989, 32(2), 351-357) oder formyliert (z. B. J. Org. Chem. 2000, 65, 7145-7150) werden. Die nachfolgende Cyclokondensation von 1,3-Dioxoverbindungen **(5)** mit Bisguanidin **(3)** erfolgt in bekannter Art und führt zu den gewünschten 2-Guanidinderivaten der Formel **IV** (Org. Lett. 2001, 3(24), 3887-3889). Generell können auch heterocyclische Oxoverbindungen der Formel **1** in analoger Weise zu den entsprechenden Zielverbindungen der Formel **IV** umgesetzt werden. Zu beachten ist dabei, dass eine in Q des Ausgangsprodukts vorhandene -NH-Gruppe mit einer gängigen Schutzgruppe versehen wird.

Typischerweise wird die Synthese sowohl der erfindungsgemässen Guanidinderivate der allgemeinen Formel **II** als auch der entsprechenden Zwischenprodukte in Lösung unter Verwendung eines organischen Lösungsmittels durchgeführt. Einführen und Entfernen von Schutzgruppen erfolgen mit typischen, dem Fachmann bekannten Methoden (T.W. Greene & P.G.M. Wuts in Protective Groups in Organic Synthesis, Third Edition, John Wiley & Sons, 1999).

Geeignete organische Lösungsmittel sind solche, welche sich unter den gewählten Reaktionsbedingungen inert verhalten. Es sind dies bevorzugt Ether, wie Diethylether, Dioxan, Tetrahydrofuran, Glycoldimethylether; oder Alkohole, zum Beispiel Methanol, Ethanol, Propanol, Isopropanol, Butanol, iso-Butanol oder tert-Butanol; oder Kohlenwasserstoffe, wie Benzol, Toluol, Xylol, Hexan, Cyclohexan oder Petroleum-Fraktionen; oder halogenierte Kohlenwasserstoffe, wie Dichlormethan, Trichlormethan, Tetrachlormethan, Dichlorethylen, Trichlorethylen oder Chlorbenzol; oder auch Ethylacetat, Triethylamin, Pyridine, Dimethylsulfoxid, Dimethylformamid, Hexamethylphosphoramid, Acetonitril, Aceton oder Nitromethan. Ebenfalls können Mischungen der erwähnten Lösungsmittel verwendet werden.

Basen, welche für die beschriebenen Prozesse verwendet werden können, sind im allgemeinen anorganische oder organische Basen. Bevorzugt werden Alkalimetallhydroxide, zum Beispiel Natrium- oder Kaliumhydroxid, Erdalkalimetallhydroxide, zum Beispiel Bariumhydroxid, Alkalimetallcarbonate wie Natriumcarbonat oder Kaliumcarbonat, Erdalkalimetallcarbonate wie Calciumcarbonat, oder Alkalimetall- oder Erdalkalimetallalkoxide wie Natrium- oder Kaliummethoxid, Natrium- oder Kaliumethoxid oder Kalium-tert-butoxid, oder organische Amine, z.B.

Trialkyl(C1-C6)-amine, wie Triethylamin, oder heterocyclische Amine, wie 1,4-Diazabicyclo[2.2.2]octan (DABCO), 1,8-Diazabicyclo[5.4.0]undec-7-en (DBU), Pyridin, 4-Dimethyl-aminopyridin, N-Methylpiperidin oder N-Methylmorpholin. Es ist auch möglich, Alkalimetalle, wie Natrium, oder deren Hydride, wie Natriumhydrid, zu verwenden. Die erwähnten Basen können, wo dienlich, als säurebindendes Hilfsmittel verwendet werden.

Als Kupplungsreagenzien können dehydratisierende Reagenzien dienen, beispielsweise Carbodiimide, wie Diisopropylcarbodiimid, Dicyclohexylcarbodiimid oder N-(3-Dimethylaminopropyl)-N'-ethylcarbodiimid-hydrochlorid, oder Cärbonylverbindungen, wie Carbonyldiimidazol, oder 1,2-Oxazolium-Verbindungen, wie 2-Ethyl-5-phenyl-isoxazolium-3-sulfonat, oder auch Propanphosphonsäureanhydrid oder iso-Butylchloroformiat oder Benzotriazolyloxy-tris-(dimethylamino)phosphonium-hexafluorophosphat (BOP) oder Diphenylphosphoramidat oder Methansulfonylchlorid, wenn angebracht in Gegenwart von Basen, wie Triethylamin oder N-Ethylmorpholin oder N-Methylpiperidin oder Diisopropylethylamin.

Die nachfolgenden Beispiele sollen zur Erläuterung der vorliegende Erfindung dienen, diese aber in keiner Weise einschränken.

### Beispiel 1

### N-(4-Methyl-5,6,7,8-tetrahydro-chinazolin-2-yl)-guanidin-Carbonat

2-Acetylcyclohexanon (500 µmol, Aldrich) wurde zusammen mit Bisguanidin **3** (1 mmol) und Kaliumcarbonat (2.5 mmol) in EtOH (2 ml) vorgelegt und a) im Mikrowellenofen (10 min, 120 °C) oder b) bei 80 °C über Nacht umgesetzt. Nach beendeter Reaktion wurde das Reaktionsgemisch mit Wasser versetzt, bis alles Carbonat gelöst war, und das über Nacht ausgefallene Produkt abfiltriert. *t*_{R} 1.39; MS (pos. Ion.) *m*/*z* 206.37 [M+H]⁺.

### Bisguanidin-Carbonat 3 (Reagens für Beispiel 1)

Ein Gemisch aus Dicyandiamid (476 mmol), Ammoniumchlorid (12 mol) und Phenol (120 g) wurde 6 Stunden auf 120-140 °C erhitzt. Zur Aufarbeitung wurde das Reaktionsgemisch auf Wasser (500 ml) gegeben und zur Entfernung des Phenols mehrfach mit Diethylether extrahiert. Das Produkt wurde durch Zugabe von gesättigter Natriumcarbonatlösung ausgefällt und abfiltriert. Nach Umkristallisation aus Methanol erhielt man **3** in Form des Carbonatsalzes als fast farblosen Feststoff. (Org. Lett. 2001, 3(24), 3887-3889).

Analog zur Herstellung von Beispiel 1 wurden ausgehend von den entsprechenden cyclischen α-Acylketonen die Verbindungen gemäss Beispielen 2-18 in Tabelle 2 hergestellt. In denjenigen Fällen, in denen das Produkt nicht auskristallisierte, wurde eine chromatographische Aufreinigung an Kieselgel (Eluent: Ethylacetat/ Aceton/Wasser/ Essigsäure 16:2:1:1) durchgeführt und das Produkt entsprechend als Acetat isoliert. Sowohl die Carbonate als auch die Acetate konnten durch Lösen in methanolischer HCl und anschliessendes Entfernen des Lösungsmittels im Vakuum in die entspechenden HCl-Salze überführt werden.

In Tabelle 2 sind für die Produkte gemäss Beispielen 1-18 die Strukturformeln (einschliesslich der Säuren, von welchen sich die Anionen der erhaltenen Salze ableiten), die Namen der entsprechenden Basen sowie deren Summenformeln und Molekulargewichte, die für die Herstellung verwendeten Ausgangsprodukte sowie physikalische Daten angegeben. Bei sämtlichen Produkten handelt es sich um Racemate.

Die verwendeten cyclischen α-Acylketone sind kommerziell erhältlich oder wurden ausgehend vom entsprechenden Cycloalkanon durch Acylierung nach literaturbekannten Methoden hergestellt (J. Med. Chem. 1989, 32(2), 351-357; J. Org. Chem. 2000, 65(21), 7145-7150; J. Med. Chem. 1971, 14(10), 997-998). Nachfolgend sind für die verschiedenen Verbindungsklassen exemplarische Verfahren beschrieben.

### rac-2-Acetyl-4-phenyl-cyclohexanon (Ausgangsprodukt für Beispiel 3)

Zu einer Suspension von NaH (20 mmol) in absolutem Ethylacetat (20 mmol) wurde eine Lösung von 4-Phenylcyclohexanon (10 mmol, Lancaster) in Benzol (5 ml) getropft und das Reaktionsgemisch nach beendeter Gasentwicklung für 3 h bei 40° C gerührt. Anschliessend wurde mit Wasser versetzt, das Reaktionsgemisch dreimal mit Ether extrahiert, die vereinigten organischen Phasen mit Wasser und gesättigter Natriumchloridlösung gewaschen, über Natriumsulfat getrocknet und das Lösungsmittel im Vakuum entfernt. Nach säulenchromatographischer Aufreinigung an Kieselgel mit Hexan/EtOAc 15:1 erhielt man sauberes Produkt. *t*_{R} 2.14; MS (pos. Ion.) *m*/*z* 217.26 [M+H]⁺. (J. Med. Chem. 1989, 32(2), 351-357).

In analoger Weise wurden auch die Ausgangsprodukte für die Beispiele 4-14 in Tabelle 2 hergestellt und ohne chromatographische Reinigung als Rohprodukte gemäss dem für Beispiel 1 beschriebenen Verfahren umgesetzt.

### rac-3-Acetyl-4-oxo-piperidin-1-carbonsäure-tert-butylester (Ausgangsprodukt für Beispiel 15)

Eine Lösung von 4-Oxo-piperidin-1-carbonsäure-tert-butylester (2.5 mmol) in absolutem THF (1 ml) wurde bei -78 °C zu einer frisch bereiteten Lösung von LDA (2.76 mmol) in absolutem THF (2 ml) gegeben und 2 h bei dieser Temperatur gerührt. Anschliessend tropfte man in THF (1.5 ml) gelöstes Acetylimidazol (2.76 mmol) zu und rührte das Reaktionsgemisch über Nacht, wobei man auf Raumtemperatur erwärmen liess. Nach Zugabe von gesättigter Ammoniumchlorid-Lösung wurde dreimal mit Ether extrahiert, die vereinigten organischen Phasen mit Wasser und gesättigter Natriumchloridlösung gewaschen, über Natriumsulfat getrocknet und das Lösungsmittel im Vakuum entfernt. Nach säulenchromatographischer Aufreinigung an Kieselgel mit Hexan/EtOAc 5:1 erhielt man das Produkt als gelbes Öl. *t*_{R} 2.09; MS (neg. Ion.) m/z 240.41 [M-H]⁻. (J. Med. Chem. 1989, 32(2), 351-357).

Die Umsetzung zum Guanidin-Derivat erfolgte wie für Beispiel 1 beschrieben.

### rac-4-tert-Butyl-2-(2,2,2-trifluor-acetyl)-cyclohexanon (Ausgangsprodukt für Beispiel 16)

Zu einer Suspension von Natriummethoxid (6 mmol) und 4-tert-butyl-cyclohexanon (3 mmol) in absolutem Diethylether (3 ml) wurde eine Lösung von Ethyltrifluoracetat (6 mmol) in Diethylether (2 ml) getropft und das Reaktionsgemisch nach beendeter Gasentwicklung über Nacht bei Raumtemperatur gerührt. Anschliessend wurde mit Wasser versetzt, das Reaktionsgemisch dreimal mit Ether extrahiert, die vereinigten organischen Phasen mit Wasser und gesättigter Natriumchloridlösung gewaschen, über Natriumsulfat getrocknet und das Lösungsmittel im Vakuum entfernt. Das so erhaltene gelbe Öl wurde ohne weitere Aufreinigung als Rohprodukt gemäss dem für Beispiel 1 beschriebenen Verfahren mit Bisguanidin-Carbonat umgesetzt. (J. Med. Chem. 1971, 14(10), 997-998).

In analoger Weise wurden auch die Ausgangsprodukte für die Beispiele 17 und 18 in Tabelle 2 hergestellt und ohne chromatographische Reinigung als Rohprodukte gemäss dem für Beispiel 1 beschriebenen Verfahren umgesetzt.

### Analytische Methode

Die hergestellten Verbindungen wurden mittels *reverse-phase* HPLC analysiert (Retention time *t*_{R}) auf einem Waters Alliance LC, ausgerüstet mit einem MassLynx NT Massenspektrometer an einer GROM-SIL 120 ODS-4 HE HPLC-Kolonne (Teilchengrösse 3µm, Säulenlänge 30 mm, Durchmesser 2 mm) mit einem linearen Gradienten mit Wasser/ 0.06% Ameisensäure (A) und Acetonitril/0.06% Ameisensäure (B) von 5% auf 95% B in 3 min. mit einer Flussrate von 0.3 ml/min.

**Tabelle 2: Analytische Daten der Produkte von Beispielen 1-18**

| **Bsp.** | **Struktur** | **Name** | **Summenformel Molekulargewicht** | **Ausgangsprodukt** | **t_{R} [min]** | **MS Daten m/z [M+H]⁺** |
|---|---|---|---|---|---|---|
| 1 | 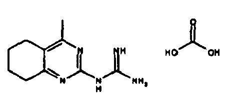 | N-(4-Methyl-5,6,7,8-tetrahydro-chinazolin-2-yl)-guanidin | C10H15N5 205.3 | Cyclohexanon | 1.39 | 206.3 |
| 2 | 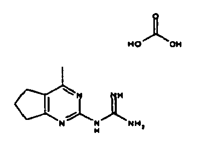 | N-(4-Methyl-6,7-dihydro-5H-cyclopentapyrimidin-2-yl)-guanidin | C9H13N5 191.2 | Cyclopentanon | 1.27 | 192.33 |
| 3 | 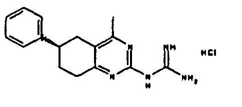 | N-(4-Methyl-6-phenyl-5,6,7,8-tetrahydro-chinazolin-2-yl)-guanidin | C16H19N5 281.4 | 4-Phenyl-cyclohexanon | 1.49 | 282.34 |
| 4 | 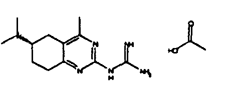 | N-(6-Isopropyl-4-methyl-5,6,7,8-tetrahydro-chinazolin-2-yl)-guanidin | C13H21N5 247.3 | 4-Isopropyl-cyclohexanon | 1.52 | 248.53 |
| 5 | 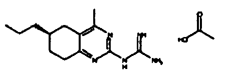 | N-(4-Methyl-6-propyl-5,6,7,8-tetrahydro-chinazolin-2-yl)-guanidin | C13H21N5 247.3 | 4-n-Propyl-cyclohexanon | 1.57 | 248.59 |
| 6 | 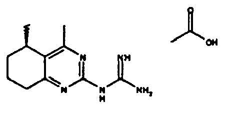 | N-(4,5-Dimethyl-5,6,7,8-tetrahydro-chinazolin-2-yl)-guanidin | C11H17N5 219.3 | 3-Methyl-cyclohexanon | 1.38 | 220.28 |
| 7 | 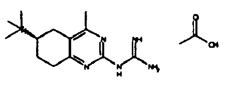 | N-(6-tert-Butyl-4-methyl-5,6,7,8-tetrahydro-chinazolin-2-yl)-guanidin | C14H23N5 261.4 | 4-tert-Butyl-cyclohexanon | 1.63 | 262.33 |
| 8 | 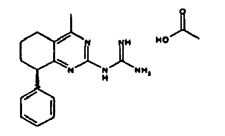 | N-(4-Methyl-8-phenyl-5,6,7,8-tetrahydro-chinazolin-2-yl)-guanidin | C16H19N5 281.4 | 2-Phenyl- cyclohexanon | 1.48 | 282.34 |
| 9 | 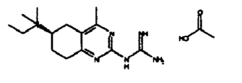 | N-[6-(1,1-Dimethylpropyl)-4-methyl-5,6,7,8-tetrahydro-chinazolin-2-yl]-guanidin | C15H25N5 275.4 | 4-tert-Amyl-cyclohexanon | 1.68 | 276.62 |
| 10 | 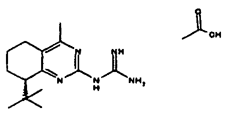 | N-(8-tert-Butyl-4-methyl-5,6,7,8-tetrahydro-chinazolin-2-yl)-guanidin | C14H23N5 261.4 | 2-tert-Butyl-cyclohexanon | 1.58 | 262.33 |
| 11 | 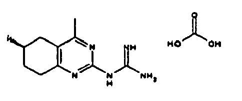 | N-(4,6-Dimethyl-5,6,7,8-tetrahydro-chinazolin-2-yl)-guanidin | C11H17N5 219.3 | 4-Methyl-cyclohexanon | 1.36 | 220.43 |
| 12 | 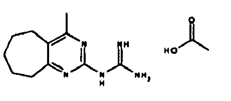 | N-(4-Methyl-6,7,8,9-tetrahydro-5H-cycloheptapyrimidin-2-yl)-guanidin | C11H17N5 219.3 | Cyclo-heptanon | 1.36 | 220.37 |
| 13 | 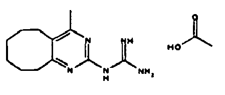 | N-(4-Methyl-5,6,7,8,9,10-hexahydro-cyclooctapyrimidin-2-yl)-guanidin | C12H19N5 233.3 | Cyclooctanon | 1.39 | 234.54 |
| 14 | 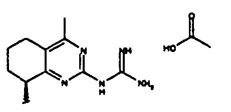 | N-(4,8-Dimethyl-5,6,7,8-tetrahydro-chinazolin-2-yl)-guanidin | C11H17N5 219.3 | 2-Methyl-cyclohexanon | 1.33 | 220.4 |
| 15 | 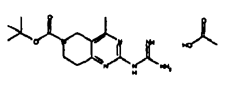 | 2-Guanidino-4-methyl-7,8-dihydro-5H-pyrido[4,3-d]pyrimidin-6-carbonsäure-tert-butylester | C14H22N6O2 piperidin-1306.4 | 4-Oxo-carbonsäure-tert-butylester | 1.47 | 307.35 |
| 15 | 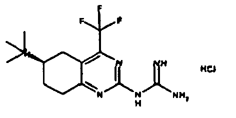 | N-(6-tert-Butyl-4-trifluoromethyl-5,6,7,8- tetrahydro-chinazolin-2-yl)-guanidin | C14H20F3N5 315.3 | 4-tert-Butyl-cyclohexanon | 1.75 | 316.4 |
| 16 | 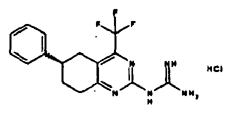 | N-(6-Phenyl-4-trifluormethyl-5,6,7,8-tetrahydro-chinazolin-2-yl)-guanidin | C16H16F3N5 335.3 | 4-Phenyl-cyclohexanon | 1.68 | 336.35 |
| 17 | 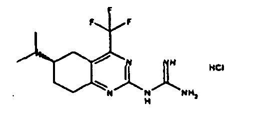 | N-(6-Isopropyl-4-trifluormethyl-5,6,7,8-tetrahydro-chinazolin-2-yl)-guanidin | C13H18F3N5 301.3 | 4-Isopropyl-cyclohexanon | 1.69 | 302.35 |

## Patentansprüche

1. Guanidinderivate der allgemeinen Formel 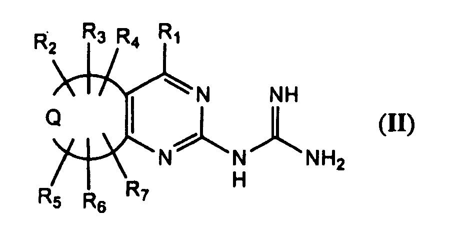 worin
R₁ Methyl, Ethyl, Trifluormethyl, Methylamino, Ethylamino, Isopropylamino, Cyclopropylamino, Methoxy, Ethoxy, Trifluormethoxy, Methylsulfanyl, oder Ethylsulfanyl bedeutet;
Q eine Kette von 3-6 gegebenenfalls substituierten C-Atomen bedeutet, wovon eines oder mehrere durch -N(R')-, -O- oder -S(O)ₘ ersetzt sein können, wobei im Falle mehrerer solcher Atome oder Gruppen diese gleich oder verschieden sein können, wobei Q zusammen mit dem Pyrimidinring ein Chinazolin-, Cyclopentapyrimidin-, Cycloheptapyrimidin, Pyridopyrimidin-, Pyranopyrimidin-, Thiopyranopyrimidin-, Pyrimidoazepin- oder Cyclooctapyrimidingerüst bildet, welches nur die drei Doppelbindungen des Pyrimidinbausteins enthält;
R' Methyl, Ethyl, Propyl, Hexyl, 2,2-Dimethylpropionyl, Cyclopropylmethyl, 2-Cyclohexylethyl, Propinyl, Ethyloxycarbonylethyl, Benzyl, n-Butyloxycarbonyl, tert-Butyloxycarbonyl, Benzyloxycarbonyl, 3-Methyl-butyryl, Pentanoyl, Phenylacetyl, 2-Propylpentanoyl, Cyclopropancarbonyl, Isobutyryl, But-3-enoyl, 2-Methoxyacetyl, Propane-2-sulfonyl, Butane-1-sulfonyl, Methansulfonyl, tert-Butyloxycarbonylaminopropionyl oder 4-Dimethylamino-butyryl bedeutet;
R₂ Methyl, Ethyl, n-Propyl, Isopropyl, n-Butyl, Isobutyl, tert.-Butyl, 1,1-Dimethylpropyl oder Phenyl bedeutet; R₃-R₇ Wasserstoff, Methyl, Ethyl, n-Propyl, i-Propyl, n-Bütyl oder t-Butyl bedeuten; und
m 0, 1 oder 2 bedeutet;
pharmazeutisch verwendbare Säureadditionssalze von basischen Verbindungen der Formel **II,** pharmazeutisch verwendbare Salze von sauren Gruppen enthaltenden Verbindungen der Formel **II** mit Basen, pharmazeutisch verwendbare Ester von Hydroxy- oder Carboxygruppen enthaltenden Verbindungen der Formel **II** sowie Hydrate oder Solvate davon.

2. Verbindungen gemäss Anspruch 1, worin Q zusammen mit einem Pyrimidinring ein 6,7-Dihydro-5H-cyclopentapyrimidin-, 5,6,7,8-Tetrahydrochinazolin-, 6,7,8,9-Tetrahydro-5H-cycloheptapyrimidin-, 5,6,7,8,9,10-Hexahydrocyclooctapyrimidin-, 6,7-Dihydro-5H-pyrrolopyrimidin- oder 5,6,7,8-Tetrahydropyridopyrimidingerüst bildet.

3. Verbindungen gemäss Anspruch 1, wobei die Guanidinderivate der Formel **II** von den folgenden Verbindungen ausgewählt wird:
- N-(4-Methyl-5,6,7,8-tetrahydro-chinazolin-2-yl)-guanidin;
- N-(4-Methyl-6,7-dihydro-5H-cyclopentapyrimidin-2-yl)-guanidin;
- N-(4-Methyl-6-phenyl-5,6,7,8-tetrahydro-chinazolin-2-yl)-guanidin;
- N-(6-Isopropyl-4-methyl-5,6,7,8-tetrahydro-chinazolin-2-yl)-guanidin;
- N-(4-Methyl-6-propyl-5,6,7,8-tetrahydro-chinazolin-2-yl)-guanidin;
- N-(4,5-Dimethyl-5,6,7,8-tetrahydro-chinazolin-2-yl)-guanidin;
- N-(6-tert-Butyl-4-methyl-5,6,7,8-tetrahydro-chinazolin-2-yl)-guanidin;
- N-(4-Methyl-8-phenyl-5,6,7,8-tetrahydro-chinazolin-2-yl)-guanidin;
- N-[6-(1,1-Dimethyl-propyl)-4-methyl-5,6,7,8-tetrahydro-chinazolin-2-yl]-guanidin;
- N-(8-tert-Butyl-4-methyl-5,6,7,8-tetrahydro-chinazolin-2-yl)-guanidin;
- N-(4,6-Dimethyl-5,6,7,8-tetrahydro-chinazolin-2-yl)-guanidin;
- N-(4-Methyl-6,7,8,9-tetrahydro-5H-cycloheptapyrimidin-2-yl)-guanidin;
- N-(4-Methyl-5,6,7,8,9,10-hexahydro-cyclooctapyrimidin-2-yl)-guanidin;
- N-(4,8-Dimethyl-5,6,7,8-tetrahydro-chinazolin-2-yl)-guanidin;
- 2-Guanidino-4-methyl-7,8-dihydro-5H-pyrido[4,3-d]pyrimidin-6-carbonsäure-tert-butylester;
- N-(6-tert-Butyl-4-trifluoromethyl-5,6,7,8-tetrahydro-chinazolin-2-yl)-guanidin;
- N-(6-Phenyl-4-trifluormethyl-5,6,7,8-tetrahydro-chinazolin-2-yl)-guanidin; und
- N-(6-Isopropyl-4-trifluormethyl-5,6,7,8-tetrahydro-chinazolin-2-yl)-guanidin.

4. Verbindungen gemäss einem der Ansprüche 1-3 zur Anwendung als therapeutische Wirkstoffe.

5. Arzneimittel, enthaltend eine Verbindung gemäss einem der Ansprüche 1-3 und einen inerten Träger.

6. Verwendung von Verbindungen gemäss einem der Ansprüche 1-3 als Neuropeptid FF Rezeptor-Antagonisten zur Herstellung eines Arzneimittels zur Behandlung von Schmerz und Hyperalgesie, von Entzugserscheinungen bei Alkohol-, Psychopharmaka- und Nicotinabhängigkeit und zur Verhinderung oder Aufhebung dieser Abhängigkeiten, zur Regulierung der Insulin-Freisetzung, der Nahrungsaufnahme, von Gedächtnisfunktionen, des Blutdrucks, des Elektrolyt- und Energiehaushaltes und zur Behandlung von Harninkontinenz.

## Claims

1. Guanidine derivatives of general formula 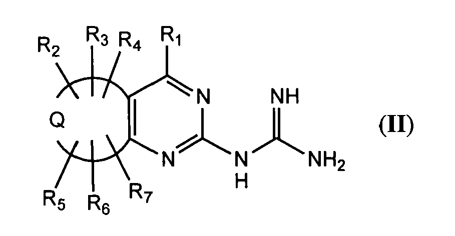 in which
R₁ means methyl, ethyl, trifluoromethyl, methylamino, ethylamino, isopropylamino, cyclopropylamino, methoxy, ethoxy, trifluoromethoxy, methylsulphanyl or ethylsulphanyl;
Q means a chain of 3-6 optionally substituted C atoms, one or more of which can be replaced by -N(R')-, -O- or -S(O)ₘ, in the case of many such atoms or groups these being able to be identical or different, wherein Q, together with the pyrimidine ring, forms a quinazoline, cyclopentapyrimidine, cycloheptapyrimidine, pyridopyrimidine, pyranopyrimidine, thiopyranopyrimidine, pyrimidoazepine or cyclooctapyrimidine skeleton, which contains only the three double bonds of the pyrimidine component;
R' means methyl, ethyl, propyl, hexyl, 2,2-dimethylpropionyl, cyclopropylmethyl, 2-cyclohexylethyl, propinyl, ethyloxycarbonylethyl, benzyl, n-butyloxycarbonyl, tert-butyloxycarbonyl, benzyloxycarbonyl, 3-methylbutyryl, pentanoyl, phenylacetyl, 2-propylpentanoyl, cyclopropanecarbonyl, isobutyryl, but-3-enoyl, 2-methoxyacetyl, propane-2-sulphonyl, butane-1-sulphonyl, methanesulphonyl, tert-butyloxycarbonylaminopropionyl or 4-dimethylaminobutyryl; R₂ means methyl, ethyl, n-propyl, isopropyl, n-butyl, isobutyl, tert.-butyl, 1,1-dimethylpropyl or phenyl;
R₃-R₇ mean hydrogen, methyl, ethyl, n-propyl, i-propyl, n-butyl or t-butyl; and
m means 0, 1 or 2;
pharmaceutically acceptable acid addition salts of basic compounds of formula **II,** pharmaceutically acceptable salts of acid group-containing compounds of formula **II** with bases, pharmaceutically acceptable esters of hydroxy or carboxy group-containing compounds of formula **II** as well as hydrates or solvates thereof.

2. Compounds according to claim 1, in which Q together with a pyrimidine ring forms a 6,7-dihydro-5H-cyclopentapyrimidine, 5,6,7,8-tetrahydro-quinazoline, 6,7,8,9-tetrahydro-5H-cycloheptapyrimidine, 5,6,7,8,9,10-hexahydrocyclooctapyrimidine, 6,7-dihydro-5H-pyrrolopyrimidine or 5,6,7,8-tetrahydropyridopyrimidine skeleton.

3. Compounds according to claim 1, the guanidine derivatives of formula **II** being chosen from the following compounds:
- N-(4-methyl-5,6,7,8-tetrahydro-quinazolin-2-yl)-guanidine;
- N-(4-methyl-6,7-dihydro-5H-cyclopentapyrimidin-2-yl)-guanidine;
- N-(4-methyl-6-phenyl-5,6,7,8-tetrahydro-quinazolin-2-yl)-guanidine;
- N-(6-isopropyl-4-methyl-5,6,7,8-tetrahydro-quinazolin-2-yl)-guanidine;
- N-(4-methyl-6-propyl-5,6,7,8-tetrahydro-quinazolin-2-yl)-guanidine;
- N-(4,5-dimethyl-5,6,7,8-tetrahydro-quinazolin-2-yl)-guanidine;
- N-(6-tert-butyl-4-methyl-5,6,7,8-tetrahydro-quinazolin-2-yl)-guanidine;
- N-(4-methyl-8-phenyl-5,6,7,8-tetrahydro-quinazolin-2-yl)-guanidine;
- N-[6-(1,1-dimethyl-propyl)-4-methyl-5,6,7,8-tetrahydro-quinazolin-2-yl]-guanidine;
- N-(8-tert-butyl-4-methyl-5,5,7,8-tetrahydro-quinazolin-2-yl)-guanidine;
- N-(4,6-dimethyl-5,6,7,8-tetrahydro-quinazolin-2-yl)-guanidine;
- N-(4-methyl-6,7,8,9-tetrahydro-5H-cycloheptapyrimidin-2-yl)-guanidine;
- N-(4-methyl-5,6,7,8,9,10-hexahydro-cyclooctapyrimidin-2-yl)-guanidine;
- N-(4,8-dimethyl-5,6,7,8-tetrahydro-quinazolin-2-yl)-guanidine;
- 2-guanidino-4-methyl-7,8-dihydro-5H-pyrido[4,3-d]pyrimidine-6-carboxylic acid tert-butyl ester;
- N-(6-tert-butyl-4-trifluoromethyl-5,6,7,8-tetrahydro-quinazolin-2-yl)-guanidine;
- N-(6-phenyl-4-trifluoromethyl-5,6,7,8-tetrahydro-quinazolin-2-yl)-guanidine; and
- N-(6-isopropyl-4-trifluoromethyl-5,6,7,8-tetrahydro-quinazolin-2-yl)-guanidine.

4. Compounds according to one of claims 1-3 for use as therapeutic active ingredients.

5. A medicinal product containing a compound according to one of claims 1-3 and an inert carrier.

6. Use of compounds according to one of claims 1-3 as neuropeptide FF receptor-antagonists for the preparation of a medicinal product for the treatment of pain and hyperalgesia, of withdrawal symptoms in the case of alcohol, psychotropics and nicotine dependence and for the prevention or elimination of these dependences, for the regulation of insulin secretion, food intake, memory functions, blood pressure, and of the electrolyte and energy balance and for the treatment of urinary incontinence.

## Revendications

1. Dérivés de la guanidine de formule générale 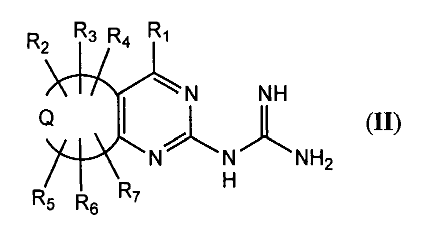 dans laquelle
R₁ signifie méthyle, éthyle, trifluorométhyle, méthylamino, éthylamino, isopropylamino, cyclopropylamino, méthoxy, éthoxy, trifluorométhoxy, méthylsulfanyle ou éthylsulfanyle ;
Q signifie une chaîne de 3-6 atomes de C éventuellement substitués, dont un ou plusieurs peut ou peuvent être remplacés par -N(R')-, -O- ou -S(O)ₘ, étant entendu que dans le cas où plusieurs de ces atomes ou groupements sont présents ceux-ci peuvent être identiques ou différents, de sorte que Q forme ensemble avec le cycle pyrimidine un squelette de quinazoline, cyclopentapyrimidine, cycloheptapyrimidine, pyridopyrimidine, pyranopyrimidine, thiopyranopyrimidine, pyrimidoazépine ou cyclooctapyrimidine, qui ne contient que les trois liaisons doubles du composant pyrimidine ;
R' signifie méthyle, éthyle, propyle, hexyle, 2,2-diméthylpropionyle, cyclopropylméthyle, 2-cyclohexyléthyle, propinyle, éthyloxycarbonyléthyle, benzyle, n-butyloxycarbonyle, tert-butyloxycarbonyle, benzyloxycarbonyle, 3-méthylbutyryle, pentanoyle, phénylacétyle, 2-propylpentanoyle, cyclopropanecarbonyle, isobutyryle, but-3-ènoyle, 2-méthoxyacétyle, propane-2-sulphonyle, butane-1-sulphonyle, méthanesulphonyle, tert-butyloxycarbonylaminopropionyle ou 4-diméthylaminobutyryle; R₂ signifie méthyle, éthyle, n-propyle, isopropyle, n-butyle, isobutyle, tert.-butyle, 1,1-diméthylpropyle ou phényle;
R₃-R₇ signifient hydrogène, méthyle, éthyle, n-propyle, i-propyle, n-butyle ou t-butyle et m signifie 0, 1 ou 2 ;
sels pharmaceutiquement acceptables d'addition d'acides sur des composés basiques de formule **II,** sels pharmaceutiquement acceptables de composés de formule **II** contenant des groupements acides avec des bases, esters pharmaceutiquement acceptables de composés de formule **II** contenant des groupements hydroxy ou carboxy ainsi que des hydrates ou des produits de solvatation de ces derniers.

2. Composés selon la revendication 1, dans lesquels Q, ensemble avec un cycle pyrimidine, forme un squelette 6,7-dihydro-5H-cyclopentapyrimidine, 5,6,7,8-tétrahydroquinazoline, 6,7,8,9-tétrahydro-5H-cycloheptapyrimidine, 5,6,7,8,9,10-hexahydrocyclooctapyrimidine, 6,7-dihydro-5H-pyrrolopyrimidine ou 5,6,7,8-tétrahydropyridopyrimidine.

3. Composés selon la revendication 1, les dérivés de la guanidine de formule **II** étant choisis parmi les composés suivants :
- N-(4-méthyl-6-5,6,7,8-tétrahydro-quinazolin-2-yl)-guanidine ;
- N-(4-méthyl-6,7-dihydro-5H-cyclopentapyrimidin-2-yl)-guanidine ;
- N-(4-méthyl-6-phényl-5,6,7,8-tétrahydro-quinazolin-2-yl)-guanidine ;
- N-(6-isopropyl-4-méthyl-5,6,7,8-tétrahydro-quinazolin-2-yl)-guanidine ;
- N-(4-méthyl-6-propyl-5,6,7,8-tétrahydro-quinazolin-2-yl)-guanidine ;
- N-(4,5-diméthyl-5,6,7,8-tétrahydro-quinazolin-2-yl)-guanidine ;
- N-(6-tert-butyl-4-méthyl-5,6,7,8-tétrahydro-quinazolin-2-yl)-guanidine.
- N-(4-méthyl-8-phényl-5,6,7,8-tétrahydro-quinazolin-2-yl)-guanidine ;
- N-[6-(1,1-diméthyl-propyl)-4-méthyl-5,6,7,8-tétrahydroquinazolin-2-yl]-guanidine ;
- N-(8-tert-butyl-4-méthyl-5,6,7,8-tétrahydro-quinazolin-2-yl)-guanidine ;
- N-(4,6-diméthyl-5,6,7,8-tétrahydro-quinazolin-2-yl)-guanidine ;
- N-(4-méthyl-6,7,8,9-tétrahydro-5H-cycloheptapyrimidin-2-yl)-guanidine ;
- N-(4-méthyl-5,6,7,8,9,10-hexahydro-cyclooctapyrimidin-2-yl)-guanidine ;
- N-(4,8-diméthyl-5,6,7,8-tétrahydro-quinazolin-2-yl)-guanidine ;
- ester tert-butylique de l'acide 2-guanidino-4-méthyl-7,8-dihydro-5H-pyrido[4,3-d]pyrimidine-6-carboxylique ;
- N-(6-tert-butyl-4-trifluorométhyl-5,6,7,8-tétrahydroquinazolin-2-yl)-guanidine ;
- N-(6-phényl-4-trifluorométhyl-5,6,7,8-tétrahydroquinazolin-2-yl)-guanidine et
- N-(6-isopropyl-4-trifluorométhyl-5,6,7,8-tétrahydroquinazolin-2-yl)-guanidine.

4. Composés selon l'une quelconque des revendications 1-3 en vue d'une utilisation en tant que constituants thérapeutiques actifs.

5. Médicament contenant un composé selon l'une quelconque des revendications 1-3 et un excipient inerte.

6. Utilisation de composés selon l'une quelconque des revendications 1-3 en tant qu'antagonistes des récepteurs de neuropeptides FF pour la préparation d'un médicament en vue du traitement des douleurs et de l'hyperalgésie, des syndromes de sevrage en cas de dépendances de l'alcool, des psychotropes et de la nicotine et en vue de la prévention ou de l'élimination de ces dépendances, en vue de la régulation de la sécrétion de l'insuline, de la prise de nourriture, des fonctions de la mémoire, de la pression sanguine et de l'équilibre électrolytique et énergétique et en vue du traitement de l'incontinence urinaire.
